Europäisches Patentamt

European Patent Office

Office européen des brevets

(19)

(11) Veröffentlichungsnummer : **0 028 725**
**B1**

(12) # EUROPÄISCHE PATENTSCHRIFT

(45) Veröffentlichungstag der Patentschrift :
18.01.84

(51) Int. Cl.³ : **C 07 C 45/42, C 07 C 47/52**

(21) Anmeldenummer : 80106301.7

(22) Anmeldetag : 16.10.80

(54) Verfahren zur Herstellung von aromatischen Aldehyden nach der Sommelet-Reaktion.

(30) Priorität : 24.10.79 DE 2942894

(43) Veröffentlichungstag der Anmeldung :
20.05.81 Patentblatt 81/20

(45) Bekanntmachung des Hinweises auf die Patenterteilung : 18.01.84 Patentblatt 84/03

(84) Benannte Vertragsstaaten :
BE DE FR GB IT NL

(56) Entgegenhaltungen :
FR-A- 2 365 547
HOUBEN-WEYL "Methoden der organischen Chemie" 4. Auflage, Band 7, Teil 1 1954, GEORG THIEME VERLAG, Stuttgart Seiten 194 bis 198

(73) Patentinhaber : BASF Aktiengesellschaft
Carl-Bosch-Strasse 38
D-6700 Ludwigshafen (DE)

(72) Erfinder : Schoch, Werner, Dr.
Neustrasse 4
D-6925 Eschelbonn (DE)
Erfinder : Kroener, Michael, Dr.
Eislebener Strasse 8
D-6800 Mannheim 42 (DE)
Erfinder : Widder, Rudi, Dr.
In der Taesch 7
D-6906 Leimen (DE)

EP 0 028 725 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von aromatischen Aldehyden nach der Sommelet-Reaktion

Die Umsetzung von Monohalogenmethylaromaten mit Urotropin, die zu aromatischen Aldehyden führt, ist eine altbekannte Reaktion, die unter dem Namen « Sommelet-Reaktion » bekannt ist.

Hierbei setzt man den Monohalogenmethylaromaten mit Urotrophin (Hexamethylentetramin) um, wobei sich intermediär das Arylalkylurotropiniumhalogenid bildet, das sich in das Salz einer Schiff'schen Base umlagert, und wobei nebenher Methylenimin entsteht, das sich durch Reaktion von Formaldehyd und Ammoniak — entstanden durch den Zerfall des Urotropins — bildet. Aus diesen beiden Molekülen entsteht schließlich durch Hydridverschiebung der Aldehyd neben Methylamin. In diesem Zusammenhang sei auf die zusammenfassende Darstellung von S. J. Angyal, Org. Reactions 8, 197 ff (1954) verwiesen. Aus diesem Artikel und auch aus anderen Lehren geht hervor, daß man als Ausgangsstoffe reine Monohalogenmethylaromaten benötigt, also beispielsweise Benzylchlorid oder p-Xylychlorid und andere, um die Reaktion als Eintopfreaktion durchführen zu können.

Im allgemeinen arbeitete man bisher in Lösungsmitteln wie Alkohol, Alkoholwassergemischen oder Eisessig.

Falls ungereinigtes Ausgangsprodukt gewählt wurde, empfahlen die früheren Autoren, das Zwischenprodukt, nämlich das Arylalkylurotropiniumhalogenid zwischenzuisolieren und dann erst die Sommelet-Reaktion ablaufen zu lassen.

Nach diesen Methoden erhält man laut Angyal loc. cit. maximale Ausbeuten von ca. 70 % der Theorie.

Da aromatische Aldehyde wichtige Zwischenprodukte darstellen, und vor allem die Homologen des Benzaldehyds mit anderen Methoden wirtschaftlich in nicht allzu befriedigender Weise zugänglich sind, stellte die Optimierung der Sommelet-Reaktion wegen der leichten Zugänglichkeit der Ausgangsprodukte ein Ziel der vorliegenden Erfindung dar.

Gegenstand der Erfindung ist ein Verfahren zur Herstellung von aromatischen Monoaldehyden in einer Eintopfreaktion durch Umsetzung von in einer Methylgruppe monohalogenierten 1, 2 oder 3 Methylgruppen enthaltenden 1- oder 2-kernigen Aromaten mit Hexamethylentetramin nach dem Schema einer Sommelet-Reaktion, wobei die monochlorierten Aromaten ein rohes Halogenierungsgemisch darstellen, saure Hydrolyse des Reaktionsgemisches und Abtrennung der gebildeten Monoaldehyde, das dadurch gekennzeichnet ist, daß man ein solches rohes Halogenierungsgemisch einsetzt, das durch Photochlorierung von Methylaromaten mit für die Bildung von Monochlorierungsprodukten unterschüssigen Mengen an Chlor entstanden ist, auf dieses Halogenierungsgemisch ohne vorherige Aufarbeitung das Hexamethylentetramin einwirken läßt und anschließend unter Zusatz von Wasser die Sommelet-Reaktion ablaufen läßt. Es wurde gefunden, daß man bei dieser Arbeitsweise — bezogen auf Monohalogen — methylaromaten-Ausbeuten von über 80 Gew.-% der Theorie an aromatischen Monoaldehyden erhält. Das Überraschende besteht darin, daß trotz des starken Anteils an Nebenprodukten letztendlich reine Produkte erhalten werden, ohne daß nennenswerte Schwierigkeiten bei der Aufarbeitung auftreten. Vor allem aber ist bemerkenswert, daß die in dem Ausgangsprodukt enthaltenen höherchlorierten Methylaromaten die Reaktion nicht stören.

Die Ausgangsgemische erhält man durch Photochlorierung von Methylaromaten. Beispielhaft zu nennen sind Toluol, p-tert.-Butyltoluol, p-Xylol oder α- und β-Methylnaphthaline. Auch Gemische sind einsetzbar, woraus dann die entsprechenden Aldehydgemische resultieren.

Bei der Photochlorierung dieser Methylaromaten geht man so vor, daß man das Chlor gegenüber der zu monohalogenierenden Methylgruppe im Unterschuß einsetzt, um mehrfache Chlorierungen möglichst zurückzudrängen, d. h. die etwaige Bildung von z. B. Benzalchlorid oder doppelt chloriertem p-Xylol möglichst hintanzuhalten. Bedingt durch den Chlorunterschuß enthält das erhaltene Chlorierungsgemisch noch bis zu 50 Gew.% an unveränderten Methylaromaten. Außerdem sind nicht zu vermeidende Mengen (2 bis 4 Gew.%) an kernsubstituierten Aromaten und 4 bis 6 Gew.% an höher in den Seitenketten chlorierten Produkten anwesend.

Die Reaktion führt man vorteilhaft in der Weise durch, daß man das Chlorierungsgemisch mit Urotropin im Molverhältnis — bezogen auf reinen Monochlormethylaromaten — von 1 : 1 bis 1 : 1,5 vereinigt und den Ansatz unter Ausschluß von Sauerstoff, zweckmäßigerweise unter Stickstoffatmosphäre, ca. 5 bis 30 Minuten mechanisch bewegt, was mit einem Rührer erfolgen kann.

Anschließend gibt man Wasser — etwa die halbe bis dreifache Gewichtsmenge — bezogen auf reinen Chlormethylaromaten — zu und erhitzt ca. 1 1/2 bis 5 Stunden unter Rückflußkühlung.

Anschließend setzt man Mineralsäuren, wie konzentrierte Salzsäure in einer Menge zu, daß die wäßrige Phase einen pH-Wert von ca. 3 bis 4 aufweist, erhitzt ca. 15 Minuten auf bis zu 100 °C, läßt erkalten und trennt dann die Wasserphase ab.

Gegebenenfalls unter Zusatz von geringen Mengen an Stabilisiermitteln, wie Hydrochinon, wird anschließend die organische Phase fraktioniert. Der erhaltene Aldehyd wird abgetrennt, unveränderter Methylaromat in die Chlorierung zurückgeführt, und die übrigen Nebenprodukte werden entweder verworfen oder anderweitig verwendet.

Die Aldehyde fallen in Ausbeuten von über 80 % der Theorie, bezogen auf Chlormethylaromaten, und in einer Reinheit von mehr als 99 % an.

Das Verfahren ist von besonderer Wichtigkeit bei der Herstellung von p-Tolylaldehyd aus p-Xylylchlorid oder von Benzaldehyd aus Benzylchlorid.

Das nun folgende repräsentative Beispiel soll die Erfindung erläutern.

Beispiel

1 050,1 g chloriertes p-Xylol, bestehend aus 47,61 % p-Xylylchlorid, 43,76 % p-Xylol, 3,6 % kernchloriertem p-Xylol und 5,03 % höherchlorierten p-Xylolen, wobei dieses Gemisch genau 500 g (3,55 Mol) an p-Xylylchlorid enthält, werden in einem 4-l-Dreihalskolben mit Rückflußkühler und Rührer mit 546,7 g (3,9 Mol) Urotropin 15 Minuten unter Stickstoff gerührt.

Anschließend gibt man 1 000 g Wasser zu und erhitzt 3 Stunden unter Rückflußkühlung. Man läßt erkalten und stellt bei Raumtemperatur mit 170 ml conc. HCl auf einem pH-Wert von 3 bis 4 ein und erhitzt noch weitere 15 Minuten unter Rückflußkühlung.

Man läßt erkalten und trennt die Wasserphase ab. Man setzt zur organischen Phase 0,01 g Hydrochinon zu und destilliert.

Man erhält 352,74 g (82,22 % der Theorie) an p-Tolylaldehyd mit einem Reinheitsgehalt von 99 %.

## Ansprüche

1. Verfahren zur Herstellung von aromatischen Monoaldehyden in einer Eintopfreaktion durch Umsetzung von in einer Methylgruppe monohalogenierten 1, 2 oder 3 Methylgruppen enthaltenden 1- oder 2-kernigen Aromaten mit Hexamethylentetramin nach dem Schema einer Sommelet-Reaktion, wobei die monochlorierten Aromaten ein rohes Halogenierungsgemisch darstellen, saure Hydrolyse des Reaktionsgemisches und Abtennung der gebildeten Monoaldehyde, dadurch gekennzeichnet, daß man ein solches rohes Halogenierungsprodukt einsetzt, das durch Photochlorierung von Methylaromaten mit für die Bildung von Monochlorierungsprodukten unterschüssigen Mengen an Chlor entstanden ist, auf dieses Halogenierungsgemisch ohne vorherige Aufarbeitung das Hexamethylentetramin einwirken läßt und anschließend unter Zusatz von Wasser die Sommelet-Reaktion ablaufen läßt.

2. Verfahren nach Anspruch 1, dadurch gekennzeichnet, daß man das rohe Photochlorierungsgemisch des p-Xylols oder Toluols einsetzt.

## Claims

1. A process for the preparation of aromatic monoaldehydes in a one-vessel process by reacting mononuclear or binuclear aromatics, which contain 1, 2 or 3 methyl groups and are monohalogenated in a methyl group, with hexamethylenetetramine by a Sommelet reaction, the monochlorinated aromatics being a crude halogenation mixture, subjecting the reaction mixture to acid hydrolysis and separating off the monoaldehydes formed, wherein the crude halogenation product used is one which has been obtained by photochlorination of methyl-aromatic with less than the stoichiometric amount of chlorine required for the formation of monochlorination products, this halogenation mixture is treated, without prior working up, with hexamethylenetetramine, and the Sommelet reaction is then carried out with the addition of water.

2. A process as claimed in claim 1, wherein the crude mixture obtained by photochlorinating p-xylene or toluene is employed.

## Revendications

1. Procédé de préparation de monoaldéhydes aromatiques, dans une réaction en un seul et même récipient, par réaction d'aromatiques à 1 ou 2 noyaux, contenant 1, 2 ou 3 groupes méthyle monohalogénés dans un groupe méthyle, avec de l'hexaméthylènetétramine, selon le schéma d'une réaction Sommelet, les aromates monochlorés représentant un mélange brut d'halogénisation, hydrolyse acide du mélange de réaction et séparation du monoaldéhyde formé, caractérisé par le fait que l'on met en œuvre un produit d'halogénisation brut tel qu'il est produit par photochloration de méthylaromates avec une quantité de chlore insuffisante pour la formation de produits de monochloration, on fait agir sur ce mélange d'halogénation, sans traitement préalable, l'hexaméthylènetétramine, puis, avec addition d'eau, on laisse se dérouler la réaction de Sommelet.

2. Procédé selon la revendication 1, caractérisé par le fait qu'on met en œuvre le mélange brut de photochloration du p-xylène ou toluène.